# EUROPEAN PATENT SPECIFICATION

(11) **EP 2 170 410 B1**
(45) Date of publication and mention of the grant of the patent: **12.06.2013**
(21) Application number: 08771976.1
(22) Date of filing: 26.06.2008
(51) Int. Cl.: A61L 15/28, A61L 15/32

(54) **COMPOSITE DEVICE FOR THE REPAIR OR REGENERATION OF TISSUE**
VERBUNDVORRICHTUNG FÜR GEWEBEREPARATUR ODER -REGENERATION
DISPOSITIF COMPOSITE POUR LA RÉPARATION OU LA RÉGÉNÉRATION D'UN TISSU

(30) Priority: 29.06.2007 US 824517
(43) Date of publication of application: 07.04.2010
(73) Proprietor: Ethicon, Incorporated, Somerville, NJ 08876 (US)
(72) Inventor: BROWN, Laura, J., Hamilton Square, NJ 08690 (US); PANOZZO, Mary, F., Fort Wayne, IN 46814 (US); WARD, James, J., Stewartsville, NJ 08886 (US); GOSIEWSKA, Anna, Skillman, NJ 08558 (US); MARSDEN, Donald, Christopher, Meadow Lane, Cononley BD20 8NY (GB)
(74) Representative: James, Anthony Christopher W.P.
(86) International application number: PCT/US2008/068265
(87) International publication number: WO 2009/006171

(56) References cited:
- WO-A-2006/034568
- GB-A- 2 314 842
- US-A- 5 676 967
- US-A1- 2006 149 182

## Description

### BACKGROUND OF THE INVENTION

### FIELD OF THE INVENTION

The invention relates to tissue-engineering scaffolds for the repair and/or regeneration of tissue. Specifically, the invention relates to a composite device comprising a tissue-engineering scaffold and a coating of ORC and collagen.

### BACKGROUND ART

Tissue-engineering scaffolds useful for wound healing are known in the art. Although these scaffolds provide a substrate to support cell ingrowth, they fail to provide an optimal stimulus to entice cell ingrowth.

Wounds are defined as a disruption of normal anatomic structure and function, which can be present internally (underlying the skin) or externally (present on the skin surface). Wounds vary in their location and in their duration (acute versus chronic) in addition to their underlying pathology.

Acute wounds represent approximately one tenth of the 2 billion wounds annually occurring in the US and Western Europe. Acute wounds typically heal through the body's normal healing response. Acute wounds include surgical wounds, such as those from plastic, cosmetic or reconstruction surgery, soft tissue defects, such as voids present after removal of tumors or other surgical excision, and wounds resulting from skin conditions, and traumatic injury.

Chronic wounds represent approximately 7-8 million of the 2 billion wounds that occur annually. Chronic wounds do not heal because the normal repair process of destroying damaged tissue and simultaneously forming new tissue is disrupted. Chronic wounds are delayed in their progression to closure, can remain open for months to years, and frequently reoccur. Chronic wounds may be either partial or full thickness in depth and may arise from a variety of pathological outcomes. Chronic wounds include diabetic, pressure, venous or arterial ulcers, non-healed surgical wounds, and wounds resulting from skin cancers, bums and the like.

Various therapies for the treatment of chronic wounds have been described. One approach involves using tissue-engineering scaffolds alone. Tissue-engineering scaffolds come in a variety of forms such as weaves, knits, braids, perforated films, meshes, non-wovens, and foams. Scaffolds for tissue-engineering are utilized to provide structure and shape, to guide developing tissues, and to allow cells to attach, proliferate, and differentiate. By definition, tissue-engineering scaffolds are three dimensional, highly porous structures that allow cell and tissue growth and transport of nutrients and waste. Once the newly formed tissue has filled the void, it is desirable to have the scaffold naturally degrade with minimal tissue response. The process of biodegradation can occur by enzymatic cleavage, by surface erosion or by hydrolytic cleavage.

Another approach involves materials and devices designed to interact with the wound and promote new tissue formation. One such device is a 55/45 weight/weight percent collagen and oxidized regenerated cellulose (collagen and ORC) matrix which is sold under the trade name PROMOGRAN (Ethicon, Inc., Somerville, NJ). In addition to enticing cells into the wound deficit, the success of PROMOGRAN may be attributed to a number of factors, most notably the ability to inactivate degradative factors characteristic of chronic wounds such as, proteases, oxygen free radicals and excess metal ions. Additionally, collagen and ORC has demonstrated the ability to sequester growth factors and deliver them back to the wound over time, promote human dermal fibroblast proliferation and human dermal fibroblast cell migration. Although the collagen and ORC promotes cell ingrowth in a chronic wound, it does not provide any sustained structural support for the attachment and proliferation of cells.

In recent years, several clinical studies, such as Zuloff-Shani, A., Kachel, E., Frenkel, O., Orenstein, A., Shinar, E., Danon, D., "Macrophage suspensions prepared from a blood unit for treatment of refractory human ulcers," Transfus Apher Sci, 2004 Apr. 30, (2) 163-7; Frenkel, O., Shani, E., Ben-Bassat, I., Brok-Simoni, F., Rozenfeld-Granot, G., Kajakaro, G., Rehavi, G., Amariglio, N., Shinar, I., Danon, D., "Activated macrophages for treating skin ulceration: gene expression in human monocytes after hypo-osmotic shock," Clin Exp Immunol. 2002 Apr. 128 (1); Danon, D., Madjar, J., Edinov, E., Knyszynski, A., Brill, S., Diamantshtein, L., Shinar, E., "Treatment of human ulcers by application of macrophages prepared from a blood unit," Exp Gerontol 1997 Nov-Dec; 32 (6); 633-41; Danon, D., Frenkel, O., Diamandstein, L., Windler, E., Orenstein, A., "Macrophage treatment of pressure sores in paraplegia," J Wound Care 1998 Jun, 7 (6)281-3 have demonstrated the utility of adding exogenous activated macrophage suspensions to help stimulate nonhealing wounds. The mechanism of action for this clinically beneficial treatment is most likely attributed to the dual role of the macrophage as both a key secretory cell and a key driver of phagocytosis. Since many chronic wounds have delayed closure associated with infection, increased presence of activated macrophages would be beneficial in the healing of chronic wounds. Macrophages, as secretory cells, initiate and potentiate the wound healing cascade by enhancing cell migration, angiogenesis, extracellular matrix deposition and epithelialization. Activated macrophages are also known to produce a variety of cytokines, platelet-derived growth factor-bb, insulin-like growth factor, epidermal growth factor, as well as transforming growth factor alpha and beta, which are well documented to facilitate the wound healing response.

GB-A-2314842 describes a composite wound dressing material consisting of a sheet of Surgicel^{®} fabric that is coated by a freeze-dried collagen sponge. The Surgicel^{®} fabric consists of oxidized regenerated cellulose (ORC).

WO-A-2006034568 describes an elastic wound dressing material formed from gelatin and a polymer which may be chitosan or an alginate. The gelatin/polymer material may be applied to supports made of various polymers such as silk or cellulose.

US-A-20060149182 relates to collagen/ORC sponge materials for use in wound dressings. The materials further comprise antimicrobial amounts of silver. The sponge materials may contain up to about 90 wt.% of textile fibres.

US-A-5676967 describes forming a coating of collagen and beta-glucan on a multifilament polyester mesh.

A need still exists for a chronic wound therapy that provides both a stimulus for cell ingrowth, and sustained structural support for cell migration and proliferation.

The present invention provides a composite device for the treatment of chronic wounds comprising, a tissue-engineering scaffold and a coating of an anionic polysaccharide and collagen, wherein: said tissue-engineering scaffold is selected from the group consisting of sponges, foams, perforated films and textiles and is formed of a biocompatible, biodegradable aliphatic polyester; and said anionic polysaccharide is oxidized regenerated cellulose.

The scaffold is coated, i.e. the scaffold's surface is covered completely or partially, with a layer or film composition comprising oxidized regenerated cellulose and collagen. In a preferred embodiment, the composite device is capable of increasing the presence of activated macrophages in tissue or wounds. This increase can be relative to the presence of activated macrophages that would normally be found in an untreated tissue or wound and/or the presence of activated macrophages found in a tissue or wound treated with one of the following: a) a scaffold as described herein that is not coated as described herein, and b) a composition comprising ORC and collagen in the absence of such a scaffold.

The tissue-engineering scaffold is prepared from a biocompatible aliphatic polyester polymer. The biocompatible polymers used to prepare the tissue-engineering scaffold are also biodegradable. Biodegradable polymers break down over time when exposed to body tissue. Typically, the polymers degrade and are either absorbed by the body or passed through the body. Therefore, the biodegraded polymers do not elicit a chronic foreign body reaction. One skilled in the art will appreciate that the selection of a suitable material for forming the tissue-engineering scaffold depends upon several factors. These factors include the form of the scaffold, in vivo mechanical performance, cell response to the material in terms of cell attachment, proliferation, migration and differentiation, biocompatibility, and optionally, biodegradation kinetics. Other relevant factors include the chemical composition, spatial distribution of the constituents, the molecular weight of the polymer, and the degree of crystallinity.

Preferred aliphatic polyesters are selected from the group consisting of homopolymers and copolymers of lactide (which includes lactic acid, D-, L- and meso lactide), glycolide (including glycolic acid), epsilon-caprolactone, p-dioxanone (1,4-dioxan-2-one), trimethylene carbonate (1, 3-dioxan-2-one), alkyl derivatives of trimethylene carbonate, delta-valerolactone, beta-butyrolactone, gamma-butyrolactone, epsilon- decalactone, hydroxybutyrate (repeating units), hydroxyvalerate (repeating units), 1,4-dioxepan-2-one (including its dimer 1,5, 8,12-tetraoxacyclotetradecane-7,14-dione), 1,5-dioxepan-2-one, 6,6-dimethyl-1, 4-dioxan-2-one 2,5-diketomorpholine, pivalolactone, alpha, alpha- diethylpropiolactone, ethylene carbonate, ethylene oxalate, 3-methyl-1,4- dioxane-2,5-dione, 3,3-diethyl-1,4-dioxan-2,5-dione, 6,8-dioxabicycloctane-7- one and polymer blends thereof. Also preferred are biocompatible, biodegradable polymers of poly(glycolic acid-co-lactic acid). A commercially available example of this type of polymer is VICRYL^{®} (polyglactin 910 suture, Ethicon, Inc., Somerville, NJ). More preferably, the scaffold is a copolymer of glycolide and lactide having a mole ratio of glycolide to lactide of about 70:30. In this embodiment, the ratio is more preferably 80:20, and most preferably, 90:10.

The tissue-engineering scaffold can be in the form of sponges, foams, perforated films, and textiles, such as weaves, knits, braids, meshes, and non-wovens. Variables for the tissue scaffolds include either the porosity or density and scaffold thickness and dimensions. The porosity or density of the scaffold should be such that it supports cell ingrowth, attachment, and proliferation. The desired scaffold thickness and dimensions may depend upon the depth and area of the wound. Preferably, the tissue-engineering scaffold provides sustained structural support for the attachment and proliferation of cells. For example, the tissue-engineering scaffold may be present at least 7 days past implantation of the composite device. However, the scaffold is not intended to reside persistently or permanently in the body because it is biodegradable.

In one embodiment, the tissue-engineering scaffold is a textile structure including, but not limited to weaves, knits, braids, meshes, and nonwovens. The fibers used to make the textile structures can be monofilaments, yarns, threads, braids, or bundles of fibers. Also, filaments that form the fibers may be co-extruded to produce a filament with a sheath/core construction. Such filaments can be comprised of a sheath of biodegradable polymer that surrounds one or more cores comprised of another biodegradable polymer. Filaments with a fast-absorbing sheath surrounding a slower-absorbing core may be desirable in instances where extended support is necessary for tissue ingrowth. The fibers used to make the textile structures can be comprised of biocompatible, biodegradable polymers as described above.

In this embodiment, it is preferred that the tissue-engineering scaffold is a nonwoven material, such as a fabric. The fibers, as described above, are made into a nonwoven material using standard textile processing. The nonwoven fabric may be prepared from yam, scrims, netting or filaments that have been made by processes that include spinning, weaving or knitting. The yam, scrims, netting and/or filaments can be crimped to enhance entanglement with each other. Such crimped yarn, scrims, netting and/or filaments can then be cut into a staple that is long enough to entangle. The staple may be carded to create nonwoven bats, which may be then needle punched or calendared. Additionally, the staple may be kinked or piled. Other methods known for the production of nonwoven fabrics can be utilized and include such processes as air laying, wet forming and stitch bonding. Such procedures are generally discussed in the Encyclopedia of Polymer Science and Engineering, Vol. 10, pp. 204-253 (1987) and Introduction to Nonwovens by Albin Turbank (Tappi Press, Atlanta GA 1999). The density of the nonwoven scaffold is preferably from 30 to 90 milligrams/cubic centimeter. The thickness of the nonwoven scaffold is preferably from 0.2 mm to 2.2 mm. More preferably, the thickness of the nonwoven may range from 0.5 mm to 1.2 mm.

In another embodiment, the tissue-engineering scaffold is a foam. The foam tissue-engineering scaffold is prepared from biocompatible, biodegradable elastomeric copolymers. Elastomeric copolymers are materials that at room temperature can be stretched repeatedly to at least about twice its original length and upon release of stress, will return to approximately its original length. Suitable biocompatible, biodegradable elastomeric copolymers include, but are not limited to elastomeric copolymers of epsilon-caprolactone and glycolide (preferably having a mole ratio of epsilon-caprolactone to glycolide of from 30:70 to 70:30, more preferably 35:65 to 65:35); elastomeric copolymers of epsilon-caprolactone and lactide, including L-lactide, D-lactide blends thereof or lactic acid copolymers (preferably having a mole ratio of epsilon- caprolactone to lactide of from 35:65 to 65:35 and more preferably 30:70 to 45:55); elastomeric copolymers of p-dioxanone (1,4-dioxan-2-one) and lactide including L-lactide, D-lactide and lactic acid (preferably having a mole ratio of p-dioxanone to lactide of from 40:60 to 60:40); elastomeric copolymers of epsilon-caprolactone and p- dioxanone (preferably having a mole ratio of epsilon-caprolactone to p-dioxanone of from 30:70 to 70:30); elastomeric copolymers of p-dioxanone and trimethylene carbonate (preferably having a mole ratio of p-dioxanone to trimethylene carbonate of from 30:70 to 70:30); elastomeric copolymers of trimethylene carbonate and glycolide (preferably having a mole ratio of trimethylene carbonate to glycolide of from 30:70 to 70:30); elastomeric copolymers of trimethylene carbonate and lactide including L-lactide, D-lactide, blends thereof or lactic acid copolymers (preferably having a mole ratio of trimethylene carbonate to lactide of from 30:70 to 70:30) and blends thereof. Preferably, the elastomeric copolymer is epsilon-caprolactone and glycolide having a mole ratio of epsilon-caprolactone to glycolide of from about 30:70 to about 70:30. More preferably the mole ratio is from about 35:65 to about 65:35. Most preferably, the elastomeric copolymer is about 35:65 poly(epsilon-caprolactone-co-glycolide).

The foams can be prepared by processes such as lyophilization, supercritical solvent foaming (i.e., as described in EP 464,163B1), gas injection extrusion, gas injection molding or casting with an extractable material (*i.e.*, salts, sugar or any other means known to those skilled in the art). The foam can be prepared by lyophilization as described in Example 2 of U.S. Patent No. 6,712,850B2. In general, a polymer solution is prepared and poured into a mold. The mold is then transferred to a lyophilizer where the solution is frozen and then vacuum dried, thereby removing the solvent by sublimation and/or drying which results in the polymer foam. Preferably, the porosity of the foam scaffold is greater than 90 percent by volume. The foam scaffold thickness is preferably from about 0.25 mm to about 0.75 mm. More preferably, the thickness of the foam is from about 0.4 mm to about 0.6 mm.

a Oxidized cellulose is any material produced by the oxidation of cellulose for example with dinitrogen tetroxide. Such oxidation partiaiiy or completely converts primary alcohol groups on the saccharide residues to carboxylic acid groups, forming uronic acid residues within the cellulose chain. The oxidation generally does not proceed with complete selectivity, and as a result hydroxyl groups on carbons 2 and 3 are occasionally converted to the keto form. These keto units introduce an alkali label link, which at pH 7 or higher initiates the decomposition of the polymer via formation of a lactone and sugar ring cleavage. Preferably, the oxidized cellulose has an average molecular weight greater than 50,000, and is biodegradable and bioabsorbable under physiological conditions. Preferably, the oxidized cellulose is not neutralized. However, the present invention encompasses the use of partially or completely neutralized materials as described in EP0437095 B1.

ORC can be prepared by oxidation of regenerated cellulose such as rayon. An example of ORC is the commercially available haemostatic product, SURGICEL (Registered Trade Mark of Johnson & Johnson Medical, Inc.). This product is produced by the oxidation of a knitted rayon material. ORC is also commercially available as INTERCEED (Registered Trade Mark of Johnson & Johnson Medical, Inc.), an ORC fabric product which was shown to reduce the extent of post-surgical adhesions in abdominal surgery. Useful forms of ORC include particles, such as fiber particles or powder particles. Specifically, the ORC fibers, are obtained for example, by milling an ORC fabric, such as those described above. The milled ORC fibers are from about 0.5 to about 1.5 millimeters in length.

The composite device comprises a coating of ORC and collagen, including the known types of native and recombinant collagens. When a recombinant collagen is used, it is preferred that it is a human recombinant collagen of any of the known types. Certain complexes between structural proteins and anionic polysacchardies are described in US4614794 A and GB2314842 A. The coating of ORC and collagen as described herein is a film or layer over the surface of the scaffold. The coating can cover the surface of the scaffold partially or fully. Thus, the coating can be a continuous film or layer over the entire scaffold, or it can be a film or layer over the scaffold surface in a particular area or section of the scaffold. The coating can be present on predominately one side of a multi-dimensional scaffold. Alternatively, the coating can be present on all sides of a multi-dimensional scaffold. Preferably, the coating infiltrates or covers substantially all of the surface area of the scaffold, which may contain pores, interstitial spaces, channels, apertures, cavities or voids. The thickness of the coating can vary. More than one coating can be present on the scaffold. Further, the coating may be uniform in thickness over its entire area or its thickness can vary. Preferably, the ORC and collagen has a mole ratio of ORC to collagen from about 10:90 to about 90:10.

The device of the invention may be used in a kit comprising the sterilized composite device of the invention. Preferably, the kit comprises one or more sterile packages, each of which contains the sterile composite device. Most preferably, the kit comprises one or more packages, each of which contains more than one sterile composite device. The composite devices contained in the single package can have any form, shape or size, for example, foam, sponge, films or textiles ranging in any shape or size. A package that contains composite device(s) that are of a relatively small size compared to a wound size to be treated is convenient for treating a wound with the right amount of the material without having to modify the shape or size of the devices to fit the wound.

The device of the invention may be used in a method of treating a wound comprising, a) contacting a wound with one or more composite devices of the invention, b) allowing said devices and said wound to remain in contact with each other, and c) covering or closing said wound, wherein said wound is treated. The wound is closed by any means capable of sealing or mechanically connecting the edges of the wound together. Alternatively, the wound can simply be covered with any appropriate shield, film, dressing, ointment or salve. As described herein, the wounds can include any interruption of the continuity of a tissue. Preferably, the tissue is skin. More preferably, the wound is a chronic wound. Chronic wounds include diabetic, pressure, venous or arterial ulcers, non-healed surgical wounds, and wounds resulting from skin cancers, burns and the like. The wound to be treated is contacted with one or more composite devices of the present invention. The wound and the device(s) are allowed to remain in contact.

The composite device can be prepared by the following method which is more fully described in Example 1. A collagen and ORC slurry is prepared by first dissolving collagen in acetic acid and then adding ORC fibers to the solution. The mixture is homogenized in a blender and then placed in a vacuum oven and degassed. The degassed collagen and ORC mixture is poured into a mold. A tissue-engineering scaffold, as described herein, is fully immersed in the slurry. The mold containing the scaffold and the collagen and ORC slurry is frozen, more collagen and ORC slurry is added to the mold, and frozen again. The frozen scaffold and the collagen and ORC may then be lyophilized to obtain the composite device. The infiltration of the collagen and ORC into the tissue-engineering scaffold can be controlled by utilising any of a number of techniques known in the art, such as placing the material in a vacuum oven. The composite device may be provided in the form of a sheet or in the form of pieces. Pieces may be obtained by cutting the composite device sheets into pieces in any useful size or shape, for example in the size of approximately 1-4 mm in length and width and having a polygon shape. Either the sheet or pieces may then be packaged and provided sterilized and ready for use.

The composite device described herein provides both structural support for cell migration and proliferation and a stimulus for cell ingrowth. The composite device is especially suitable for the repair of chronic wounds. Chronic wounds may be either partial or full thickness in depth and may arise from a variety of pathological outcomes. Chronic wounds include diabetic, pressure, venous or arterial ulcers, non-healed surgical wounds, and wounds resulting from skin cancers, bums and the like.

### EXAMPLES

### Example 1

### Preparation of composite devices and controls

The following composite devices in the form of sheets were prepared. A composite device comprising a nonwoven scaffold coated with collagen and ORC, and a composite device comprising a foam scaffold coated with collagen and ORC. Controls include the nonwoven scaffold alone, the foam scaffold alone, and the foam of collagen and ORC alone.

### PGA and PLA Nonwoven scaffold preparation

90/10 mole/mole percent poly(glycolic acid-co-lactic acid) (PGA/PLA) nonwoven scaffolds having a density of 67.5 milligrams /cubic centimeter and 1.86 mm thickness were used. The nonwoven scaffold was scoured by immersing in isopropyl alcohol (IPA) and sonicated for about 30 minutes. The IPA was decanted off and the scaffold was rinsed with deionized (DI) water three times. The nonwoven scaffold was then immersed in DI water and sonicated for about 30 minutes. The nonwoven scaffold was subsequently dried under a flow of dry nitrogen followed by drying under high vacuum (<150 microns) for a minimum of 30 minutes. The nonwoven scaffolds were cut into 1.5 x 1.5 centimeter squares. The scaffolds were sterilized using ethylene oxide.

### PCL and PGA Foam scaffold preparation

A 5 weight/volume percent solution of 35/65 mole/mole percent poly (epsilon-caprolactone-co-glycolic acid) (PCL/PGA) copolymer (Ethicon, Inc. Somerville, NJ) 1,4-dioxane was prepared by adding one part polymer to every nine parts of solvent. The polymer and solvent were heated with stirring at about 60° C for approximately 4-8 hours until the polymer dissolved. The polymer solution was filtered and about 10 grams of the solution was poured into an aluminum mold approximately 4.5 inches (114 mm) square and approximately 12.7 mm deep. The polymer solution filled molds were placed on the shelf of an FTS Dura Dry Freeze dryer that was precooled to about -17°C. After about 15 minutes, the cycle was started and the temperature was held at -17°C for about 60 minutes. Vaccum was then applied to initiate primary drying of the dioxane by sublimation at approximately 100 mTorr for about 60 minutes. Next secondary drying was conducted a about 5° C for about 60 minutes and about 20° C for about 60 minutes. The vacuum was maintained at approximately 20 mTorr. The lyophilizer was brought to room temperature and the vacuum was broken with dry nitrogen. After purging with dry nitrogen for about 30 minutes, the foam was lifted from the mold. The foam scaffolds were cut into 1.5 x 1.5 centimeter squares. The scaffolds were sterilized using ethylene oxide.

### Collagen and ORC Slurry Preparation

A commercially available oxidized regenerated cellulose, SURGICEL (Ethicon Inc, Somerville, NJ) was milled using a rotary knife cutter through a screen-plate, while maintaining the temperature below about 60° C. A collagen suspension of 1.39 % solid content in water was prepared from bovine corium, split from cowhides according to the method in US6309454 B1. 197.84 g of the suspension was used to provide 2.75 g dry weight of collagen. 2.25 g of milled ORC was added to provide a 55/45 ratio of collagen to ORC solids. 0.05 M acetic acid was added up to a final volume of 500 ml and the mixture was blended in a Waring blender at low speed for 3 x 30 seconds. The slurry was then degassed in a vacuum oven for about 15 minutes.

### Collagen and ORC foam preparation

9.85 grams of the collagen and ORC slurry were poured into a petri dish. The petri dish was then placed in a freezer at about -27° C for about 45 minutes. Another 9.85 grams of the slurry were added to the petri dish and it was returned to the freezer for approximately 45 minutes at about -27 ° C. The petri dish containing the frozen collagen and ORC slurry was then lyophilized to dry the foam for about 24 hours at about -20 ° C. The collagen and ORC foam was cut into 1.5 x 1.5 centimeter squares. The foam was sterilized using ethylene oxide.

### Foam composite device and Nonwoven composite device preparation

9.85 grams of the collagen and ORC slurry were poured into a petri dish. The square scaffold (either foam or nonwoven as prepared above) was fully immersed in the slurry. The petri dish containing a scaffold and the collagen and ORC slurry, was then placed in a freezer at about -27 ° C for about 45 minutes. Another 9.85 grams of the slurry were added to the petri dish and it was returned to the freezer for approximately 45 minutes at about -27 ° C. The petri dish containing frozen scaffold/collagen/ORC slurry was then lyophilized to dry the foam for about 24 hours at about -20 ° C. The composite devices were trimmed to remove the excess collagen and ORC foam. The composite devices were sterilized using ethylene oxide.

### Example 2

### Use of composite device in a pig wound healing model

This study determined the cellular infiltration and wound healing response of composite scaffolds in a full-thickness excisional defect. Full thickness excisional wounds (1.5 x 1.5 centimeter square) were created on the dorsal region of four swine. Control groups were a Collagen and ORC foam, PCL and PGA foam scaffold, and PGA and PLA nonwoven scaffold prepared as described in Example 1. The composite device groups were foam composite device and nonwoven composite device prepared as described in Example 1. The treatment groups were randomized over the pigs and left in place throughout the study period. There was an n of 6 per treatment group.

The treatment squares were trimmed to fit the wound and covered a commercially available 2 x 2 centimeter wound dressing, NU-GEL (Johnson and Johnson Medical, Arlington, TX). All wounds were then covered with a commercially available wound dressing, BIOCLUSIVE (Johnson and Johnson Medical, Arlington, TX). Commercially available strips of self-adhering foam, Reston (3M Medical Surgical Division, St. Paul, Minnesota), were placed between sites to prevent cross-contamination due to wound fluid leakage. Sterile gauze was secured over the dorsum of the back with a commercially available plaster tape, ZONAS (Johnson and Johnson Medical, Arlington, TX). A commercially available body stockinet, SPANDAGE (Medi-Tech International Corporation, Brooklyn, New York) was used to hold the dressings in place. On day 2 post-wounding, the bandages were changed.

Tissues were harvested from the animals on day 7. The entire wound and surrounding normal skin was excised and placed in 10% neutral buffered formalin. The tissue was bisected and the cranial half was sent for histological processing.

### Example 3

### Histological Assessments

Sections were analyzed for the greatest granulation tissue height, the presence of the scaffold or composite device, and presence of activated macrophages using histological staining with Hematoxylin and Eosin and Masson's trichrome stains.

Wound healing is measured by calculating the total amount of new tissue ingrowth into the wound area. This tissue ingrowth extends both above and below the area of the native tissue. Greatest granulation tissue height above native tissue is defined as repair in the wound bed, which extends above the area of native tissue. The greatest granulation tissue height is calculated by drawing a line across the surface of a wound to "recreate" the normal skin architecture making certain to follow the native tissue-contour. Next, a perpendicular line is drawn from this line to the greatest granulation tissue height. The greatest granulation tissue height measurements are shown in Table 1.

**Table 1. Greatest Granulation Tissue Height Above Native Tissue Results**

| Treatment | Number of treatment sites | Mean Tissue Height | SEM |
|---|---|---|---|
| PGA and PLA nonwoven scaffold | 6 | 0.77 | 0.12 |
| Nonwoven composite device | 6 | 1.36 | 0.12 |
| PCL and PGA Foam | 6 | 1.05 | 0.15 |
| Foam composite device | 6 | 0.8 | 0.3 |
| Collagen and ORC | 6 | 0.8 | 0.27 |

Histological sections evaluated at day 7 post implantation demonstrated that the Collagen and ORC was not detected in the wound bed whereas both the foam and nonwoven scaffolds were present. The nonwoven composite device demonstrated a statistically significant increase in "greatest granulation tissue height above native tissue" when compared to the nonwoven scaffold control group (p= 0.0049, T-test). This response resulted in an elevated wound surface. Histological assessment showed the presence of activated macrophages associated with the scaffold. Although the foam composite device did not demonstrate a statistical difference in greatest granulation tissue height when compared to the foam scaffold control, histological evaluation showed an increased presence of activated macrophages over the controls, which initiate and potentiate the wound healing cascade by enhancing cell migration, angiogenesis, extracellular matrix deposition and epithelialization. By contrast to both composite scaffolds, the Collagen and ORC alone did not contain increased activated macrophages. The composite device provides both a stimulus for cell ingrowth and sustained structural support for cell migration and proliferation without the use of allogeneic cells or the risk of blood borne pathogens.

### Example 4

### Clinical application of composite device

Patients who will be treated with the composite device may have the device directly applied to the wound without further pretreatment. Some chronic wounds may need to optimize the wound bed for incorporation of the composite device. This is accomplished either by surgical, mechanical, chemical, autolytic methods or maggot therapy. For debridement, local anesthetic is applied as appropriate. Necrotic or non-viable tissue is removed using a variety of methods. For surgical debridement, a sharp instrument, scalpel, curette or laser is used to remove large amounts of necrotic tissue especially when infection is associated with the wound. Mechanical debridement offers a low cost means of debridement using gauze to remove dead tissue. Because this approach does not require surgical skills, it is easily accomplished by a nurse in a wound care setting. Additionally, autolytic, enzymatic or biological pre-treatment offers a non-surgical approach to wound bed preparation.

Wounds, either debrided or non-debrided, may be first washed with sterile saline prior to application of the device. The composite device is applied to a clean wound, which is free of clinical signs of infection. In some cases, the device may need to be trimmed to fit the wound bed. The device would then be subsequently covered with a non-adherent secondary dressing and appropriate other wound dressings depending on the wound type and location.

## Claims

1. A composite device for the treatment of chronic wounds comprising, a tissue-engineering scaffold and a coating of an anionic polysaccharide and collagen, wherein:
said tissue-engineering scaffold is selected from the group consisting of sponges, foams, perforated films and textiles and is formed of a biocompatible, biodegradable aliphatic polyester; and said anionic polysaccharide is an oxidized regenerated cellulose.

2. The composite device of claim 1, wherein said scaffold is a textile selected from the group consisting of weaves, knits, braids, meshes and nonwovens.

3. The composite device of claim 2, wherein said scaffold is a nonwoven comprising a copolymer of glycolide and lactide.

4. The composite device of claim 2, wherein said scaffold is a nonwoven comprising a copolymer of glycolide and lactide having a mole ratio of glycolide to lactide of about 90:10.

5. The composite device of claim 1, wherein said scaffold is a foam comprising an elastomeric copolyester.

6. The composite device of claim 5, wherein said elastomeric copolyester comprises epsilon-caprolactone and glycolide.

7. The composite device of claim 6, wherein said elastomeric copolyester comprises a mole ratio of epsilon-caprolactone to glycolide of from 30:70 to 70:30.

8. The composite device of any preceding claim, wherein said coating of oxidized regenerated cellulose and collagen has a mole ratio of oxidized regenerated cellulose to collagen from 10:90 to 90:10.

9. The composite device of claim 1, wherein said scaffold is a foam comprising an elastomeric copolymer of epsilon-caprolactone and glycolide having a mole ratio of epsilon-caprolactone to glycolide from 35:65 to 65:35, and said coating of oxidized regenerated cellulose and collagen has a mole ratio from 10:90 to 90:10.

10. The composite device of claim 1, wherein said scaffold is a foam comprising an elastomeric copolymer of epsilon-caprolactone and glycolide having a mole ratio of epsilon-caprolactone to glycolide of about 35:65, and said coating of oxidized regenerated cellulose and collagen has a mole ratio of about 45:55.

11. The composite device of claim 1, wherein said scaffold is a nonwoven comprising a copolymer of glycolide and lactide having a mole ratio of about 90:10, and said coating of oxidized regenerated cellulose and collagen has a mole ratio of about 45:55.

## Patentansprüche

1. Verbundvorrichtung zur Behandlung von chronischen Wunden, umfassend ein Gerüst für den Gewebeaufbau und eine Beschichtung aus einem anionischen Polysaccharid und Kollagen, wobei: das Gerüst für den Gewebeaufbau aus der Gruppe bestehend aus Schwämmen, Schaumstoffen, perforierten Filmen und Textilien ausgewählt ist und aus einem biokompatiblen, bioabbaubaren aliphatischen Polyester gebildet ist und es sich bei dem anionischen Polysaccharid um eine oxidierte Regeneratcellulose handelt.

2. Verbundvorrichtung nach Anspruch 1, wobei es sich bei dem Gerüst um eine Textilie aus der Gruppe bestehend aus Geweben, Gestricken, Geflechten, Maschenwaren und Vliesstoffen handelt.

3. Verbundvorrichtung nach Anspruch 2, wobei es sich bei dem Gerüst um einen Vliesstoff, der ein Copolymer von Glykolid und Lactid umfasst, handelt.

4. Verbundvorrichtung nach Anspruch 2, wobei es sich bei dem Gerüst um einen Vliesstoff, der ein Copolymer von Glykolid und Lactid mit einem Molverhältnis von Glykolid zu Lactid von etwa 90:10 umfasst, handelt.

5. Verbundvorrichtung nach Anspruch 1, wobei es sich bei dem Gerüst um einen Schaumstoff, der einen elastomeren Copolyester umfasst, handelt.

6. Verbundvorrichtung nach Anspruch 5, wobei der elastomere Copolyester epsilon-Caprolacton und Glykolid umfasst.

7. Verbundvorrichtung nach Anspruch 6, wobei der elastomere Copolyester ein Molverhältnis von epsilon-Caprolacton zu Glykolid von 30:70 bis 70:30 umfasst.

8. Verbundvorrichtung nach einem der vorhergehenden Ansprüche, wobei die Beschichtung aus oxidierter Regeneratcellulose und Kollagen ein Molverhältnis von oxidierter Regeneratcellulose zu Kollagen von 10:90 bis 90:10 aufweist.

9. Verbundvorrichtung nach Anspruch 1, wobei es sich bei dem Gerüst um einen Schaumstoff, der ein elastomeres Copolymer von epsilon-Caprolacton und Glykolid mit einem Molverhältnis von epsilon-Caprolacton zu Glykolid von 35:65 bis 65:35 umfasst, handelt und die Beschichtung aus oxidierter Regeneratcellulose und Kollagen ein Molverhältnis von 10:90 bis 90:10 aufweist.

10. Verbundvorrichtung nach Anspruch 1, wobei es sich bei dem Gerüst um einen Schaumstoff, der ein elastomeres Copolymer von epsilon-Caprolacton und Glykolid mit einem Molverhältnis von epsilon-Caprolacton zu Glykolid von etwa 35:65 umfasst, handelt und die Beschichtung aus oxidierter Regeneratcellulose und Kollagen ein Molverhältnis von etwa 45:55 aufweist.

11. Verbundvorrichtung nach Anspruch 1, wobei es sich bei dem Gerüst um einen Vliesstoff, der ein Copolymer von Glykolid und Lactid mit einem Molverhältnis von etwa 90:10 umfasst, handelt und die Beschichtung aus oxidierter Regeneratcellulose und Kollagen ein Molverhältnis von etwa 45:55 aufweist.

## Revendications

1. Dispositif composite pour le traitement de plaies chroniques, comprenant un échafaudage de génie tissulaire et un revêtement d'un polysaccharide anionique et de collagène, **caractérisé en ce que** :
ledit échafaudage de génie tissulaire est sélectionné dans le groupe constitué par les éponges, les mousses, les films perforés et les textiles et est formé d'un polyester aliphatique biocompatible et biodégradable ; et ledit polysaccharide anionique est une cellulose oxydée et régénérée.

2. Dispositif composite selon la revendication 1, **caractérisé en ce que** ledit échafaudage est un textile sélectionné dans le groupe constitué par les tissés, les tricots, les tresses, les mailles et les non tissés.

3. Dispositif composite selon la revendication 2, **caractérisé en ce que** ledit échafaudage est un non tissé comprenant un copolymère de glycolide et de lactide.

4. Dispositif composite selon la revendication 2, **caractérisé en ce que** ledit échafaudage est un non tissé comprenant un copolymère de glycolide et de lactide ayant un rapport molaire de glycolide au lactide d'environ 90:10.

5. Dispositif composite selon la revendication 1, **caractérisé en ce que** ledit échafaudage est une mousse comprenant un copolyester élastomère.

6. Dispositif composite selon la revendication 5, **caractérisé en ce que** ledit copolyester élastomère comprend de l'epsilon-caprolactone et du glycolide.

7. Dispositif composite selon la revendication 6, **caractérisé en ce que** ledit copolyester élastomère comprend un rapport molaire d'epsilon-caprolactone au glycolide allant de 30:70 à 70:30.

8. Dispositif composite selon l'une quelconque des revendications précédentes, **caractérisé en ce que** ledit revêtement de cellulose oxydée et régénérée et de collagène présente un rapport molaire de cellulose oxydée et régénérée au collagène allant de 10:90 à 90:10.

9. Dispositif composite selon la revendication 1, **caractérisé en ce que** ledit échafaudage est une mousse comprenant un copolymère élastomère d'epsilon-caprolactone et de glycolide ayant un rapport molaire d'epsilon-caprolactone au glycolide allant de 35:65 à 65:35, et ledit revêtement de cellulose oxydée et régénérée et de collagène présente un rapport molaire allant de 10:90 à 90:10.

10. Dispositif composite selon la revendication 1, **caractérisé en ce que** ledit échafaudage est une mousse comprenant un copolymère élastomère d'epsilon-caprolactone et de glycolide ayant un rapport molaire d'epsilon-caprolactone au glycolide d'environ 35:65, et ledit revêtement de cellulose oxydée et régénérée et de collagène présente un rapport molaire d'environ 45:55.

11. Dispositif composite selon la revendication 1, **caractérisé en ce que** ledit échafaudage est un non tissé comprenant un copolymère de glycolide et de lactide ayant un rapport molaire d'environ 90:10, et ledit revêtement de cellulose oxydée et régénérée et de collagène présente un rapport molaire d'environ 45:55.
